# EUROPÄISCHE PATENTSCHRIFT

(11) **EP 1 279 672 B1**
(45) Veröffentlichungstag und Bekanntmachung des Hinweises auf die Patenterteilung: **15.12.2004**
(21) Anmeldenummer: 02015588.3
(22) Anmeldetag: 15.07.2002
(51) Int. Cl.: C07D 301/12, C07D 303/32, C07D 303/36, C07D 303/48, C07D 405/04

(54) **Polyaminosäure-katalysiertes Verfahren zur enantioselektiven Epoxidierung von alpha,beta-ungesättigten Enonen und alpha,beta-ungesättigten Sulfonen**
Process for enantioselective epoxidation of alpha, beta-unsaturated enones and alpha, beta-unsaturated sulfones catalysed by polyamino acids
Procédé pour l'époxydation enantiosélective d'énones alpha, béta-insaturées et de sulfones alpha, béta-insaturées, catalysé par des acides polyaminés

(30) Priorität: 27.07.2001 DE 10136132
(43) Veröffentlichungstag der Anmeldung: 29.01.2003
(73) Patentinhaber: Bayer Chemicals AG, 51368 Leverkusen (DE)
(72) Erfinder: Geller, Thomas, Dr., 51373 Leverkusen (DE); Krüger, Christa Maria, Dr., 48149 Münster (DE); Militzer, Hans-Christian, Dr., 51519 Odenthal (DE)

(56) Entgegenhaltungen:
- EP-A- 0 403 252
- EP-A- 1 006 111
- EP-A- 1 006 127
- WO-A-96/33183
- ADGER B.M. ET AL: "Improved procedure for Julia-Colonna asymmetric epoxidation of alpha, beta-unsaturated ketones. " JOURNAL OF THE CHEMICAL SOCIETY, PERKIN TRANSACTIONS 1., Nr. 23, - 7. Dezember 1997 (1997-12-07) Seiten 3501-3507, XP002219130 CHEMICAL SOCIETY. LETCHWORTH., GB ISSN: 0300-922X
- BAURES P.W. ET AL: "An efficient asymmetric synthesis of substituted phenyl glycidic esters" TETRAHEDRON LETTERS., Bd. 31, Nr. 45, 1990, Seiten 6501-6504, XP002006755 ELSEVIER SCIENCE PUBLISHERS, AMSTERDAM., NL ISSN: 0040-4039
- JULIA S. ET AL: "Synthetic enzymes. Part 2. Catalytic asymmetric epoxidation by means of polyamino-acids in a triphase system" JOURNAL OF THE CHEMICAL SOCIETY, PERKIN TRANSACTIONS 1., Nr. 6, 1982, Seiten 1317-1324, XP002006756 CHEMICAL SOCIETY. LETCHWORTH., GB ISSN: 0300-922X
- DHANDA, ANUPMA ET AL: "PaaSiCats: Novel polyamino acid catalysts" CHIRALITY (2000), 12(5/6), 313-317 , XP008009815
- FLOOD R.W. ET AL: "Efficient asymmetric epoxidation of alpha, beta-unsaturated ketones using a soluble triblock polyethyleneglycol-polyamino acid catalyst" ORGANIC LETTERS., Bd. 3, Nr. 5, 8. März 2001 (2001-03-08), Seiten 683-686, XP002219131 ACS, WASHINGTON, DC., US ISSN: 1523-7060

## Beschreibung

Die Erfindung betrifft ein neues Polyaminosäure-katalysiertes Verfahren zur enantioselektiven Epoxidierung von α,β-ungesättigten Enonen und α,β-ungesättigten Sulfonen unter zweiphasigen Bedingungen in Gegenwart spezieller Co-Katalysatoren.

Chiral, nicht-racemische Epoxide sind als wertvolle Bausteine für die Herstellung von optisch aktiven Wirkstoffen und Materialien bekannt (z.B. a) *Bioorg. Med*. *Chem.,* **1999**, *7*, 2145-2156; b) *Tetrahedron Lett.,* **1999**, *40,* 5421-5424). Diese Epoxide können durch enantioselektive Epoxidierung von Doppelbindungen hergestellt werden. Hierbei werden zwei Stereozentren in einem synthetischen Schritt aufgebaut. Es ist daher nicht überraschend, daß eine Vielzahl von Methoden entwickelt wurde, um Doppelbindungen enantioselektiv zu epoxidieren. Für neue, verbesserte Methoden zur enantioselektiven Epoxidierung besteht allerdings weiterhin ein großer Bedarf.

Unter den Epoxidierungsmethoden, die jeweils auf spezielle Substrate limitiert sind, finden sich auch Methoden zur enantioselektiven Epoxidierung von α,β-ungesättigten Enonen.

So wurde beispielsweise der Einsatz von chiral, nicht-racemischen Phasentransferkatalysatoren auf Alkaloidbasis zur Epoxidierung von Enonen in *Tetrahedron Lett.,* **1998**, *39*, 7563-7566, *Tetrahedron Lett.,* **1998**, *39*, 1599-1602 und *Tetrahedron Lett.,* **1976**, *21*, 1831-1834 beschrieben.

In *Tetrahedron Lett.,* **1998**, *39,* 7353-7356, *Tetrahedron Lett.,* **1998**, *39*, 7321-7322 und *Angew. Chem., Int. Ed. Engl.,* **1997**, *36,* 410-412 werden ferner Möglichkeiten zur metallkatalysierten asymmetrischen Epoxidierung von Enonen mittels organischer Hydroperoxide beschrieben.

In WO-A-99/52886 wird ferner beschrieben, dass eine enantioselektive Epoxidierung von Enonen in Gegenwart von Katalysatoren möglich ist, die auf Zuckern basieren. Eine weitere Methode zur Epoxidierung unter Verwendung von Zn-Organylen und Sauerstoff in Gegenwart eines Ephedrin-Derivats wurde in *Liebigs Ann.*/*Recueil*, **1997**, 1101-1113 veröffentlicht.

In *Angew. Chem., Int. Ed. Engl*., **1980**, *19*, 929-930, *Tetrahedron*, **1984**, *40,* 5207-5211 und *J. Chem. Soc., Perkin Trans*. 1, 1982, 1317-24 wird die sogenannte klassische dreiphasige Epoxidierungsmethode von Juliá beschrieben. Hierbei wird die enantioselektive Epoxidierung von α,β-ungesättigten Enonen unter Zusatz von enantiomeren- und diastereomerenangereicherten Polyaminosäuren in Gegenwart von wässriger Wasserstoffperoxid- und NaOH-Lösung sowie eines Aromaten bzw. halogenierten Kohlenwasserstoffs als Lösungsmittel durchgeführt. Weiterentwicklungen dieser sogenannten 3-phasigen-Bedingungen finden sich in *Org. Synth.; Mod. Trends, Proc. IUPAC Symp. 6th.,* **1986**, 275. Die Methode wird heute allgemein als Juliá-Colonna-Epoxidierung bezeichnet.

Gemäß EP-A-0 403 252 ist es möglich, bei dieser Juliá-Colonna-Epoxidierung an Stelle der ursprünglichen Lösungsmittel vorteilhafterweise auch aliphatische Kohlenwasserstoffe einzusetzen.

Gemäß WO-A-96/33183 gelingt es ferner in Gegenwart des Phasentransferkatalysators Aliquat® 336 ([(CH₃)(C₈H₁₇)₃ N⁺]Cl⁻) und unter gleichzeitiger Verwendung von in Wasser schwerlöslichem Natriumperborat an Stelle von Wasserstoffperoxid, den Bedarf an Base (NaOH) gegenüber den Bedingungen von ursprünglichen Bedingungen von Juliá und Colonna (*Tetrahedron*, **1984**, *40,* 5207-5211) von ca. 3.7 auf 1 Äquivalent zu vermindern.

Trotz dieser Verbesserungen haben die 3-phasigen-Bedingungen deutliche Nachteile. Die Reaktionszeiten liegen nach den Originalbedingungen selbst für reaktive Substrate im Bereich von Tagen. Für *trans*-Chalkon werden in Abhängigkeit von der verwendeten Polyaminosäure beispielsweise 1 - 6 Tage benötigt (*Tetrahedron*, **1984**, *40*, 5207-5211). Eine im Reaktionsgefäß ausgeführte Voraktivierung der Polyaminosäure, indem man für 12-48 h im Lösungsmittel unter Zusatz von NaOH-Lösung rührt, verkürzt die Reaktionszeit vieler Substrate auf 1 - 3 Tage. Hierbei ist keine Zwischenaufarbeitung des Katalysators erforderlich (EP-A-0 403 252). In Gegenwart des Systems NaOH/Wasserstoffperoxid kann die Voraktivierungszeit auf minimal 6 h verringert werden (*J*. *Chem. Soc., Perkin Trans*. 1, **1995**, 1467-1468)

Trotz dieser Verbesserung kann die dreiphasige Methode nicht auf Substrate angewandt werden, welche gegen Hydroxid-Ionen empfindlich sind (*J. Chem. Soc., Perkin Trans*. 1, **1997**, 3501-3507). Ein weiterer Nachteil dieser klassischen Bedingungen besteht darin, daß die Polyaminosäure während der Reaktion (bzw. bereits während der Voraktivierung) ein Gel bildet. Dieses schränkt die erwünschte Durchmischung während der Reaktion ein und erschwert die Aufarbeitung des Reaktionsgemisches.

Aus *Tetrahedron Lett.,* **2001**, 42, 3741-43 ist es bekannt, dass unter den 3-phasigen-Bedingungen der Zusatz des Phasentransferkatalysators (PTC) Aliquat 336 bei der Epoxidierung des Phenyl-*E*-Styrylsulfons nur zu einer geringen Reaktionsgeschwindigkeit (Reaktionszeit: 4 Tage) und einem schlechtem Enantiomerenüberschuß (21% ee) führt. Es ist bisher kein Beispiel für die Verwendung von PTCs zur Epoxidierung von α,β-ungesättigten Enonen unter den klassischen 3-phasigen Juliä-Colonna-Bedingungen bekannt.

Die Juliä-Colonna-Epoxidierung wurde durch eine Änderung der Reaktionsführung weiter verbessert. Gemäß *Chem. Commun.,* **1997**, 739-740 können durch Verwendung von THF, 1,2 Dimethoxyethan, *tert*.-Butylmethylether oder Ethylacetat als Lösungsmittel, einer nicht-nucleophilen Base (z.B. DBU) und des Harnstoff Wasserstoffperoxid-Komplexes als Oxidationsmittel (pseudo)-wasserfreie Reaktionsbedingungen verwirklicht werden. Unter diesen sogenannten 2-phasigen Reaktionsbedingungen erfolgt die Epoxidierung deutlich schneller. Auf diesem Wege ist daher gemäß *J. Chem. Soc., Perkin Trans*. 1, **1997**, 3501-3507 erstmals auch die enantioselektive Epoxidierung von Hydroxid-empfindlichen Enonen unter den Juliä-Colonna-Bedingungen möglich.

Als deutlicher Nachteil erweist sich jedoch die Beobachtung, dass die Polyaminosäure bei Verwendung der 2-phasigen Bedingungen in einem separaten Verfahren voraktiviert werden muß, um schnelle Reaktionszeiten und hohe Enantiomerenüberschüsse zu erreichen. Für diese Voraktivierung, die durch Rühren der Polyaminosäure in einer Toluol/NaOH-Lösung erfolgt, werden mehrere Tage benötigt. Gemäß Tetrahedron Lett., **1998**, 39, 9297-9300 erhält man dann den benötigten aktivierten Katalysator nach einer Wasch- und Trockenprozedur. Diese so aktivierte Polyaminosäure bildet jedoch unter den 2-phasigen-Bedingungen eine Paste, welche die Durchmischung während der Reaktion sowie die nachfolgende Aufarbeitung erschwert. Gemäß EP-A-1 006 127 kann dieses Problem durch eine Adsorption der aktivierten Polyaminosäure an einen festen Träger gelöst werden. Auf Silicagel geträgerte Polyaminosäuren werden als SCAT (silica adsorbed catalysts) bezeichnet.

Gemäß EP-A-1 006 111 besteht eine weitere Variante der Juliá-Colonna-Epoxidierung darin, dass die aktivierte Polyaminosäure in Gegenwart von Wasser, einem wassermischbaren Lösungsmittel (z.B. 1,2-Dimethoxyethan) und Natriumpercarbonat die enantioselektive Epoxidierung katalysiert. Aufgrund der Verwendung wassermischbarer Lösungsmittel gestaltet sich jedoch bei diesem Verfahren die Aufarbeitung (Extraktion) umständlich.

Bei der Juliá-Colonna-Epoxidierung hängt die Reaktionsgeschwindigkeit und der erreichbare Enantiomerenüberschuß (ee) stark von der verwendeten Polyaminosäure und der Art ihrer Herstellung ab (Chirality, **1997**, 9, 198-202). Um etwa vergleichbare Ergebnisse zu erhalten, wird zur Entwicklung und Beschreibung neuer Methoden in der Literatur durchgängig ein Standardsystem mit Poly-L-leucin (pll) als Katalysator und *trans*-Chalkon als Edukt verwendet. Neben D- oder L-Polyleucin werden jedoch auch andere Polyaminosäuren wie beispielsweise D- oder L-Neopentylglycin mit Erfolg verwendet (EP-A- 1 006 127).

Die Aufgabe der vorliegenden Erfindung bestand darin, ein Verfahren bereitzustellen, welches die Homo-Polyaminosäure-katalysierte enantioselektive Epoxidierung von α,β-ungesättigten Enonen und α,β-ungesättigten Enonen Sulfonen ermöglicht, aber nicht den Nachteilen der oben beschriebenen Varianten der Juliä-Colonna-Epoxidierung unterliegt. Insbesondere sollte eine schnelle, breit anwendbare Methode gefunden werden, welche die separate, zeitaufwendige und umständliche Voraktivierung der Polyaminosäure vermeidet. Gleichzeitig sollte das Verfahren Vorteile hinsichtlich der Raum/Zeit-Ausbeute, der Handhabbarkeit, der Ökonomie und Ökologie im technischen Maßstab bringen.

Überraschenderweise wurde nun gefunden, dass man die Epoxidierung von α,β-ungesättigten Enonen und α,β-ungesättigten Sulfonen unter zweiphasigen Bedingungen in Gegenwart einer Polyaminosäure als Katalysator durchführen kann, die keiner vorherigen separaten Aktivierung unterzogen wurde, wenn die Epoxidierung in Anwesenheit eines Phasentransferkatalysators erfolgt. Diese Durchführung ermöglicht überraschenderweise sehr geringe Reaktionszeiten bei gleichzeitig hohen Enantiomerenüberschüssen.

Gegenstand der Erfindung ist somit ein Verfahren zur Epoxidierung von α,β-ungesättigten Enonen oder α,β-ungesättigten Sulfonen in Gegenwart
(1) eines organischen Lösungsmittels,
(2) einer Base,
(3) eines Oxidationsmittels,
(4) einer nicht separat voraktivierten diastereomeren- und enantiomerenangereicherten Homo-Polyaminosäure als Katalysator,
   dadurch gekennzeichnet, dass die Epoxidierung
(5) in Gegenwart eines Phasentransferkatalysators und ohne Zusatz von Wasser durchgeführt wird.

Entscheidend ist, dass das erfindungsgemäßen Verfahren in Gegenwart eines Phasentransferkatalysators durchgeführt wird: Verwendbar sind beispielsweise quartäre Ammoniumsalze, quartäre Phosphoniumsalze, Onium-Verbindungen oder Pyridiniumsalze.

Bewährt haben sich vor allem quartäre Ammonium- oder Phosphoniumsalze der allgemeinen Formel (I)

(R¹R²R³R⁴A)⁺X⁻ (I)

worin
- A: für N oder P steht,
- X⁻: für ein anorganisches oder organisches Anion steht,
- R¹, R², R³ und R⁴: gleich oder verschieden sind und für Alkyl-, Aryl-, Aralkyl-, Cycloalkyl- oder Heteroaryl-Reste stehen, die durch einen oder mehrere, gleiche oder verschiedene Halogenreste substituiert sein können, oder aber jeweils zwei Reste unter Einbindung von A einen C₄-C₆-Cycloalkyl-Ring bilden können.

Bewährt haben sich solche Phasentransferkatalysatoren der allgemeinen Formel (I), bei denen A und X⁻ die oben genannten Bedeutungen besitzen und R¹, R², R³ und R⁴ gleich oder verschieden sind und für C₁-C₁₈-Alkyl, C₆-C₁₈-Aryl, C₇-C₁₉-Aralkyl, C₅-C₇-Cycloalkyl oder C₃-C₁₈-Heteroaryl stehen.

Besonders geeignet sind ((C₄H₉)₄N)⁺Hal⁻, insbesondere ((C₄H₉)₄N)⁺Br⁻, ((C₄H₉)₄P)⁺Hal⁻, insbesondere ((C₄H₉)₄P)⁺Br⁻, ((C₄H₉)₄N)⁺HSO₄⁻, ((C₈H₁₇)₄N)⁺Br⁻, [(CH₃)(C₈H₁₇)₃N⁺]Cl⁻ und [(CH₃)(C₄H₉)₃ N⁺]Cl⁻ als Phasentransferkatalysatoren.

In der allgemeinen Formel (I) steht X für ein anorganisches oder organisches Kation, bevorzugt steht X für F⁻, Cl⁻, Br⁻, I⁻, OH⁻, HSO₄⁻, SO₄⁻, NO₃⁻, CH₃COO⁻, CF₃COO⁻, C₂H₅COO⁻, C₃H₇COO⁻, CF₃SO₃⁻ oder C₄F₉SO₃⁻

Die erfindungsgemäß einzusetzenden Phasentransferkatalysatoren sind üblicherweise käuflich erhältlich oder aber nach dem Fachmann geläufigen Methoden herstellbar.

Die Menge des zugesetzten Phasentransferkatalysators ist nicht kritisch und liegt üblicherweise im Bereich von 0.1 - 20 mol%, bevorzugt im Bereich von 0,5-15 mol%, besonders bevorzugt im Bereich von 0,5-11 mol%, jeweils bezogen auf das eingesetzte α,β-ungesättigte Enon oder α,β-ungesättigte Sulfon. Bei Mengen, die noch geringer sind als 0,1 mol%, ist jedoch zu beobachten, dass bei unverändert hohem Enantiomerenüberschuss die Reaktionsgeschwindigkeit deutlich abnimmt.

Als α,β-ungesättigte Enone oder α,β-ungesättigte Sulfone können Verbindungen der allgemeinen Formel (II) eingesetzt werden 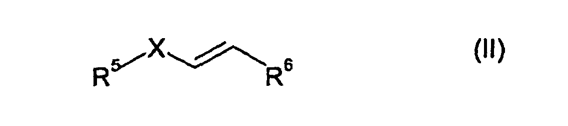 worin
- X: für (C=O) oder (SO₂) steht und
- R⁵ und R⁶: gleich oder verschieden sind und (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₇-C₁₉)-Aralkyl, (C₁-C₁₈)-Heteroaryl oder (C₂-C₁₉)-Heteroaralkyl bedeuten,
wobei die für R⁵ und R⁶ genannten Reste einfach oder mehrfach mit gleichen oder verschiedenen Resten R⁷, Halogen, NO₂, NR⁷R⁸, PO₀₋₃R⁷R⁸, SO₀₋₃R⁷, OR⁷, CO₂R⁷, CONHR⁷ oder COR⁷ substituiert sein können und gegebenenfalls eine oder mehrere CH₂-Gruppen in den Resten R⁵ und R⁶ durch O, SO₀₋₂, NR⁷ oder PO₀₋₂R⁷ substituiert sind,
wobei R⁷ und R⁸ gleich oder verschieden sind und H, (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₁-C₁₈)-Heteroaryl, (C₁-C₈)-Alkyl-(C₆-C₈)-Aryl, (C₁-C₈)-Alkyl-(C₁-C₁₉)-Heteroaryl, (C₁-C₈)-Alkyl-(C₃-C₈)-Cycloalkyl bedeuten und diese Reste R⁷ und R⁸ einfach oder mehrfach mit gleichen oder verschiedenen Halogenresten substituiert sein können.

Unter einem (C₁-C₁₈)-Alkylrest wird im Rahmen der Erfindung ein Rest mit 1 bis 18 gesättigten C-Atomen verstanden, der beliebige Verzweigungen aufweisen kann. Insbesondere sind unter dieser Gruppe die Reste Methyl, Ethyl, *n*-Propyl, Isopropyl, *n*-Butyl, Isobutyl, *sec*-Butyl, *tert*-Butyl, Pentyl und Hexyl subsumierbar.

Ein (C₂-C₁₈)-Alkenylrest weist die für den (C₁-C₁₈)-Alkylrest genannten Merkmale auf, wobei innerhalb des Restes mindestens eine Doppelbindung vorhanden sein muss.

Ein (C₂-C₁₈)-Alkinylrest weist die für den (C₁-C₁₈)-Alkylrest genannten Merkmale auf, wobei innerhalb des Restes mindestens eine Dreifachbindung vorhanden sein muss.

Unter einem (C₃-C₈)-Cycloalkylrest wird ein cylischer Alkylrest mit 3 bis 8 C-Atomen und gegebenenfalls beliebiger Verzweigung verstanden. Insbesondere zählen hierzu Reste wie Cyclopropyl, Cyclobutyl, Cyclopentyl, Cyclohexyl und Cycloheptyl. In diesem Rest kann eine oder mehrere Doppelbindungen vorhanden sein.

Unter einem (C₆-C₁₈)-Arylrest wird ein aromatischer Rest mit 6 bis 18 C-Atomen verstanden. Insbesondere zählen hierzu Reste wie Phenyl-, Naphthyl-, Anthryl- und Phenanthryl.

Unter einem (C₇-C₁₉)-Aralkylrest wird ein über einen (C₁-C₈)-Alkylrest an das Molekül gebundener (C₆-C₁₈)-Arylrest verstanden.

Ein (C₁-C₁₈)-Heteroarylrest bezeichnet im Rahmen der Erfindung ein fünf-, sechsoder siebengliedriges aromatisches Ringsystem mit 1 bis 18 C-Atomen, welches ein oder mehrere Heteroatome, bevorzugt N, O oder S im Ring aufweist. Zu diesen Heteroarylresten zählen z.B. 1-, 2-, 3-Furyl, 1-, 2-, 3-Pyrrol, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, 1-, 3-, 4-, 5-Triazolyl, 1-, 4-, 5-Tetrazolyl, Acridinyl, Chinolinyl, Phenanthridinyl, 2-, 4-, 5-, 6-Pyrimidinyl und 4-, 5-, 6-, 7-(1-Aza)-indolizinyl.

Unter einem (C₂-C₁₉)-Heteroaralkylrest wird ein dem (C₇-C₁₉)-Aralkylrest entsprechendes heteroaromatisches System verstanden.

Unter Halogen oder auch Hal versteht man im Kontext dieser Erfindung Fluor, Chlor, Brom und Iod.

Als Substrate des erfindungsgemäßen Verfahrens werden bevorzugt α,β-ungesättigte Enone oder α,β-ungesättigte Sulfone der allgemeinen Formel (II) eingesetzt, in denen
- R⁵ und R⁶: gleich oder verschieden sind und (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei die zuvor genannten Reste einfach oder mehrfach mit gleichen oder verschiedenen Resten R⁷, Halogen, NO₂, NR⁷R⁸, PO₀₋₃R⁷R⁸ oder OR⁷ substituiert sein können und R⁷ und R⁸ die für die allgemeine Formel (II) zuvor angegebene Bedeutungen besitzen.

Besonders bevorzugt werden als Substrate des erfindungsgemäßen Verfahrens α,β-ungesättigte Enone oder α,β-ungesättigte Sulfone der allgemeinen Formel (II) eingesetzt, in denen
- R⁵ und R⁶: gleich oder verschieden sind und (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei die zuvor genannten Reste einfach oder mehrfach mit gleichen oder verschiedenen Resten R⁷, Halogen, NO₂, NR⁷R⁸, PO₀₋₃R⁷R⁸ oder OR⁷ substituiert sein können und R⁷ und R⁸ die für die allgemeine Formel (II) zuvor angegebene Bedeutungen besitzen,
mit der Maßgabe, dass mindestens einer der Reste R⁵ oder R⁶ einen (C₂-C₁₂)-Alkenyl-, (C₂-C₁₂)-Alkinyl-, (C₆-C₁₂)-Aryl- oder (C₁-C₁₂)-Heteroarylrest darstellt.

Insbesondere ist es bevorzugt, Substrate der allgemeinen Formel (III) der erfindungsgemäßen Epoxidierung zu unterwerfen: 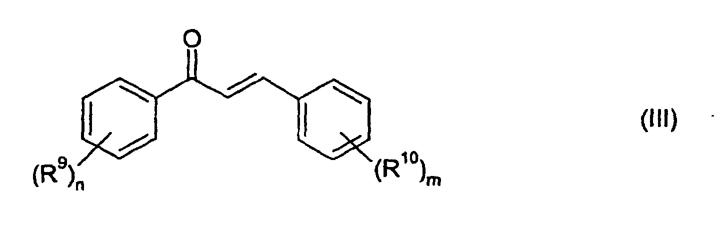 wobei
- n und m: gleich oder verschieden sind und für die Zahlen 0, 1, 2 oder 3 stehen,
und
- R⁹ und R¹⁰: gleich oder verschieden sind und NR⁷R⁸, NO₂, OR⁷, (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei diese Reste R⁹ und R¹⁰ einfach oder mehrfach mit gleichen oder verschiedenen Halogenresten substituiert sein können und R ⁷ und R⁸ die zuvor für Formel (II) genannten Bedeutungen besitzen.

Ein entscheidender Vorteil des erfindungsgemäßen Verfahrens ist die Tatsache, dass nicht separat voraktivierte Homo-Polyaminosäuren als Katalysatoren eingesetzt werden.

Für das erfindungsgemäße Verfahren können die verschiedensten diastereomerenund enantiomerenangereicherten Homo-Polyaminosäuren zum Einsatz kommen. Vorzugsweise werden allerdings Homo-Polyaminosäuren aus der Gruppe Polyneopentylglycin, Polyleucin, Polyisoleucin, Polyvalin und Polyalanin sowie Polyphenylalanin benutzt. Aus dieser Gruppe ist Polyneopentylglycin und Polyleucin am meisten zu bevorzugen.

Die Kettenlänge der Polyaminosäuren ist so zu wählen, dass einerseits die chirale Induktion bei der Reaktion nicht beeinträchtigt wird und andererseits die Kosten für die Synthese der Polyaminosäuren nicht zu groß werden. Vorzugsweise liegt die Kettenlänge der Homo-Polyaminosäuren zwischen 5 und 100, vorzugsweise 7 bis 50, Aminosäuren. Ganz besonders bevorzugt ist eine Kettenlänge von 10 bis 40 Aminosäuren.

Die Homo-Polyaminosäuren können nach Methoden des Standes der Technik hergestellt werden (*J. Org. Chem.* 1993, 58, 6247 und *Chirality* 1997, 9, 198-202). Die Methode ist auf beide optischen Antipoden der Aminosäuren anzuwenden. Der Einsatz einer bestimmten Antipode einer Polyaminosäure korreliert mit der Stereochemie des Epoxids, d.h. eine Poly-L-aminosäure führt zur optischen Antipode des Epoxids, das mit einer Poly-D-aminosäure erhalten wird.

Die Homo-Polyaminosäuren können entweder als solche unverändert in die Epoxidierung eingesetzt werden oder aber zuvor mit polyfunktionellen Aminen vernetzt oder durch andere organische Polymere kettenverlängert werden. Für eine Vernetzung setzt man vorteilhafterweise als vernetzende Amine Diaminoalkane, bevorzugt 1,3-Diaminopropan, oder quervernetztes Hydroxy- oder Aminopolystyrol (CLAMPS, kommerziell erhältlich) ein. Als Polymervergrößerer kommen bevorzugt auf Polyethylenglykol oder Polystyrol basierende Nucleophile in Frage. Derart veränderte Polyaminosäuren sind in *Chem. Commun.,* **1998**, 1159-1160 und *Tetrahedron: Asymmetry*, **1997,** *8,* 3163-3173 dargestellt.

Die Menge der eingesetzten Homo-Polyaminosäure ist nicht kritisch und liegt üblicherweise im Bereich von 0.0001 - 40 mol%, bevorzugt im Bereich von 0.001-20 mol%, besonders bevorzugt im Bereich von 0.01-15 mol%, und insbesondere im Bereich von 1 bis 15 mol-%, jeweils bezogen auf das eingesetzte α,β-ungesättigte Enon oder α,β-ungesättigte Sulfon.

Es ist auch möglich, die Homo-Polyaminosäuren in trägergebundener Form einzusetzen, was im Hinblick auf die Rückgewinnbarkeit des Katalysators und die Steigerung der optischen und chemischen Ausbeute von Vorteil sein kann.

Hierzu werden die Homo-Polyaminosäuren adsorptiv auf einem unlöslichen Trägermaterial gebunden. Als unlösliche Trägermaterialien werden vorzugweise solche auf Siliziumdioxid- oder Zeolith-Basis eingesetzt, wie z.B.Molsiebe, Silicagele, Celite® 521, Celite® Hyflo Super Cell oder Wessalith® DayP. Vorteilhaft sind auch Silicagele mit definierten Porengrößen wie z.B. CPC I oder CPC II. Weitere bevorzugte Trägermaterialien sind Aktivkohle oder Zuckerderivate wie beispielsweise Nitrocellulose und Cellulose.

Das Verhältnis von Trägermaterial zu Polyaminosäure ist durch zwei Grenzen gegeben. Einerseits kann nur eine bestimmte Anzahl von Polyaminosäure auf dem unlöslichen Träger adsorbiert werden, auf der anderen Seite läßt die chirale Induktion ab weniger als 10 Gew.% von Polyaminosäure zu Träger nach. Bevorzugt liegt das Verhältnis von Homo-Polyaminosäure zu Trägermaterial im Bereich von 1:7 bis 2:1 Gew.Teile, besonders bevorzugt im Bereich von 1:1 bis 1:4 Gew.Teile.

Das Verfahren der Trägerung ist ausführlich in EP-A-1 006 217 beschrieben, auf die hiermit ausdrücklich verwiesen wird. Hierfür wird zunächst eine Mischung aus der entsprechenden Homo-Polyaminosäure und dem Trägermaterial in einem organischen Lösungsmittel wie Ethern, z.B THF, suspendiert und anschließend für längerer Zeit, bevorzugt bis zu 48 Stunden, gerührt. Anschließend wird der Feststoff abfiltriert und getrocknet.

Sollen derartige geträgerten Katalysatoren eingesetzt werden, so eignet sich für das Verfahren der Epoxidation besonders eine Vorrichtung, die befähigt ist, nur den Katalysator zurückzuhalten. Vorzugsweise handelt es sich bei dieser Vorrichtung um einen Enzym-Membran Reaktor (C. Wandrey in Enzymes as Catalysts in Organic Synthesis; Ed.:m. Schneider, Dordrecht Riedel 1986, 263-284) Ebenso bevorzugt ist als Vorrichtung auch ein einfacher Festbettreaktor, wie z.B. eine Chromatographiesäule.

Als Oxidationsmittel dienen in der Regel Wasserstoffperoxid-Komplexe mit anorganischen Carbonaten, tertiären Aminen, Aminoxiden, Amiden, Phosphanen oder Phosphanoxiden. Bewährt hat sich besonders der Harnstoff-Wasserstoffperoxid-Komplex.

Die Menge des eingesetzten Oxidationsmittels kann in breiten Grenzen von 1-10 Äquivalenten variiert werden. Es können auch mit sehr geringen Mengen Oxidationsmittel im Bereich von 1-5 Äquivalenten, bevorzugt von 1-3 Äquivalenten und insbesondere von 1-2 Äquivalenten überraschenderweise weiterhin niedrige Reaktionszeiten und hohe Enantiomerenüberschüssen erzielt werden.

Das erfindungsgemäße Verfahren wird in Gegenwart einer Base durchgeführt, welche organischer oder anorganischer Natur sein kann. Bevorzugt werden jedoch organische, nicht-nucleophile Basen eingesetzt, insbesondere DBU (1,8-Diazabicyclo[5.4.0]undec-7-en), DBN (1,5-Diazabicyclo[4.3.0]non-5-en) oder DABCO (1,4-Diazabicyclo[2.2.2]octan).

Die Menge der eingesetzten Base kann in breiten Grenzen von 0,1-10 Äquivalenten variiert werden. Bereits mit Mengen von 0,5-5 Äquivalenten, bevorzugt von 0,8-2 Äquivalenten gelingt die erfindungsgemäße Umsetzung bei weiterhin niedrigen Reaktionszeiten und hohen Enantiomerenüberschüssen.

Das erfindungsgemäße Verfahren wird unter Einsatz eines organischen Lösungsmittels durchgeführt. Als organische Lösungsmittel kommen allgemein Ether, bevorzugt THF, Diethylether oder *tert*.-Butylmethylether, Ester, bevorzugt Ethylacetat, Amide, bevorzugt Dimethylformamid, oder Sulfoxide, bevorzugt Dimethylsulfoxid in Betracht.

Die Temperatur, die bei der Epoxidierung verwendet wird, liegt im allgemeinen im Bereich von -10 bis +50 °C, bevorzugt im Bereich von 0 bis +40 °C und insbesondere bei +10 bis +30 °C.

Im Hinblick auf die Durchführung der Reaktion werden üblicherweise alle Komponenten bis auf die Base vorgelegt und anschließend die Base zugesetzt. Es ist jedoch auch möglich, die Polyaminosäure in Gegenwart des Oxidationsmittels, der Base, des Lösungsmittels und des Phasentransferkatalysators für 15 min - 2 h zu rühren und dadurch vorzuaktivieren und anschließend ohne Zwischenisolierung der voraktivierten Homo-Polyaminosäure das zu epoxidierende Substrat zuzusetzen.

Das erfindungsgemäße 2-phasige Verfahren zur enantioselektiven Epoxidierung von α,β-ungesättigten Enonen und α,β-ungesättigten Sulfonen zeichnet sich durch die Möglichkeit zur Verwendung nicht separat voraktivierter Homo-Polyaminosäuren aus. Auf die üblicherweise notwendige zeitaufwendige (3 - 5 Tage) und umständliche separate Voraktivierung mit Zwischenisolierung kann bei diesem Verfahren aufgrund der Gegenwart eines Phasentransferkatalyators verzichtet werden. Üblicherweise werden mit dem erfindungsgemäßen Verfahren substantiell höhere Enantiomerenüberschüsse erreicht.

### Beispiele:

Der Herstellungsprozess für Polyaminosäuren liefert oft Katalysatoren für die Juliá-Colonna Epoxidierung, die eine stark unterschiedliche katalytische Aktivität aufweisen (*Chirality*, **1997**, *9*, 198-202). Der Umsatz pro Zeiteinheit und der Enantiomerenüberschuss lassen sich für ein bestimmtes Substrat nur vergleichen, wenn für die Epoxidierungsreaktion dieselbe Polyaminosäure-Charge verwendet wird. Aus diesem Grund ist ein direkter Vergleich von neuen Ergebnissen mit in der Literatur publizierten Resultaten nicht möglich, da eben zwangsläufig unterschiedliche Katalysatorchargen verwendet werden. Aus diesem Grund wurde bei allen nachfolgenden Beispielen und Vergleichsbeispielen eine einheitliche Polyleucin-Charge verwendet.

In allen nachfolgenden Beispielen wurden der Umsatz und der Enantiomerenüberschuss (*ee*-Wert) gemäß literaturbekannten Verfahren mittels HPLC an einer chiral, nicht-racemischen Phase bestimmt (UV-Detektion).

### Beispiele 1 und 3 sowie Vergleichsbeispiele 2 und 4:

### Epoxidierung von trans-Chalkon (1) zu Epoxychalkon (2) unter zweiphasigen und SCAT-Bedingungen

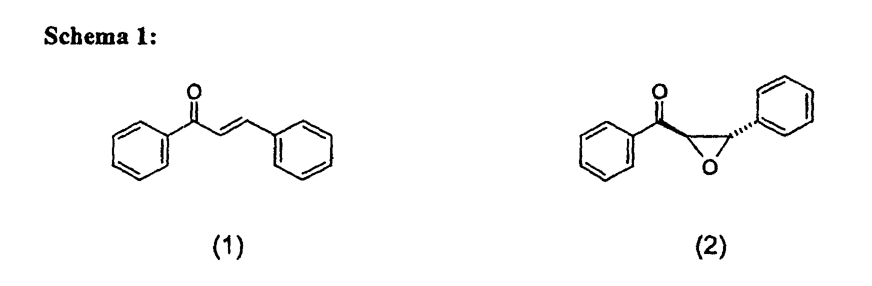

### Beispiel 1: 2-phasige Bedingungen mit PTC

50 mg *trans*-Chalkon, 35 mg Harnstoff-Wasserstoffperoxid-Komplex (UHP, 0.36 mmol, 1.5 Äquivalente), 8.5 mg [(C₄H₉)₄N⁺]Br⁻ und 100 mg nicht separat voraktiviertes pll (11 mol%) wurden gemischt, mit 1.5 ml wasserfreiem THF suspendiert und mit 55 µl DBU (1.5 Äquivalente) versetzt. Die Reaktionsmischung wurde bei Raumtemperatur unter Rühren umgesetzt. Nach 30 min Reaktionszeit wurde die Reaktionsmischung filtriert und das Filtrat und unter verringertem Druck eingeengt.

### Vergleichsbeispiel 2: 2-phasige Bedingungen ohne PTC

50 mg *trans*-Chalkon, 35 mg Harnstoff-Wasserstoffperoxid-Komplex (UHP, 0.36 mmol, 1.5 Äquivalente) und 100 mg nicht separat voraktiviertes pll (11 mol%) wurden gemischt, mit 1.5 ml wasserfreiem THF suspendiert und mit 55 µl DBU (1.5 Äquivalente) versetzt. Die Reaktionsmischung wurde bei Raumtemperatur unter Rühren umgesetzt. Nach 30 min Reaktionszeit wurde die Reaktionsmischung filtriert und das Filtrat und unter verringertem Druck eingeengt.

### Beispiel 3: SCAT-Bedingungen

### a) Herstellung von SCAT

1 g nicht separat voraktiviertes pll und 3.4 g Silicagel 60 (230-400 mesh, Merck) wurden gemischt, in 30 ml wasserfreiem THF suspendiert und unter Lichtausschluß für 48 h langsam gerührt. Die Suspension wurde filtriert und der Rückstand zweimal mit je 10 ml wasserfreiem THF gewaschen. Das Material (*SCAT*) wurde im Vakuum über P₂O₅ getrocknet.

### b) Epoxidierung unter SCAT-Bedingungen mit PTC

50 mg *trans*-Chalkon, 35 mg Harnstoff-Wasserstoffperoxid-Komplex (UHP, 0.36 mmol, 1.5 Äquivalente), 8.5 mg [(C₄H₉)₄N⁺]Br⁻ und 100 mg SCAT (11 mol%) wurden gemischt, mit 1,5 ml wasserfreiem THF suspendiert und mit 55 µl DBU (1.5 Äquivalente) versetzt. Die Reaktionsmischung wurde bei Raumtemperatur unter Rühren umgesetzt. Nach 30 min Reaktionszeit wurde die Reaktionsmischung filtriert und unter verringertem Druck eingeengt.

### Vergleichsbeispiel 4: SCAT-Bedingungen ohne PTC

### a) Herstellung von SCAT

1 g nicht separat voraktiviertes pll und 3.4 g Silicagel 60 (230-400 mesh, Merck) wurden gemischt, in 30 ml wasserfreiem THF suspendiert und unter Lichtausschluß für 48 h langsam gerührt. Die Suspension wurde filtriert und der Rückstand zweimal mit je 10 ml wasserfreiem THF gewaschen. Das Material (*SCAT*) wurde im Vakuum über P₂O₅ getrocknet.

### b) Epoxidierung unter SCAT-Bedingungen ohne PTC

50 mg *trans*-Chalkon, 35 mg Harnstoff-Wasserstoffperoxid-Komplex (UHP, 0.36 mmol, 1.5 Äquivalente) und 100 mg SCAT (11 mol%) wurden gemischt, mit 1,5 ml wasserfreiem THF suspendiert und mit 55 µl DBU (1.5 Äquivalente) versetzt. Die Reaktionsmischung wurde bei Raumtemperatur unter Rühren umgesetzt. Nach 30 min Reaktionszeit wurde die Reaktionsmischung filtriert und unter verringertem Druck eingeengt.

Die Ergebnisse der Beispiele 1 und 3 sowie der Vergleichsbeispiele VB 2 und 4 sind in der nachfolgenden Tabelle zusammengefasst.

**Tabelle:**

| Beispiel | Bedingungen | PTC | Reaktionszeit [min] | Umsatz [%] | *ee* [%] |
|---|---|---|---|---|---|
| 1 | erfindungsgemäß | [(C₄H₉)₄N⁺]Br⁻ | 30 | > 99 | 78 |
| VB 2 | 2-phasig; nicht erfindungsgemäß | ----- | 30 | > 99 | 53 |
| 3 | erfindungsgemäß | [(C₄H₉)₄N⁺]Br⁻ | 30 | > 99 | 92 |
| VB 4 | 2-phasig, SCAT; nicht erfindungsgemäß | ------ | 30 | > 99 | 86 |

## Patentansprüche

1. Verfahren zur Epoxidierung von α,β-ungesättigten Enonen oder α,β-ungesättigten Sulfonen in Gegenwart
(1) eines organischen Lösungsmittels,
(2) einer Base,
(3) eines Oxidationsmittels,
(4) einer nicht separat voraktivierten diastereomeren- und enantiomerenangereicherten Homo-Polyaminosäure als Katalysator,
**dadurch gekennzeichnet, dass** die Epoxidierung
(5) in Gegenwart eines Phasentransferkatalysators und ohne Zusatz von Wasser durchgeführt wird.

2. Verfahren nach Anspruch 1, **dadurch gekennzeichnet, dass** als Phasentransferkatalysator quartäre Ammoniumsalze, quartäre Phosphoniumsalze, Onium-Verbindungen oder Pyridiniumsalze eingesetzt werden.

3. Verfahren nach Anspruch 2, **dadurch gekennzeichnet, dass** als PhasentransferKatalysatoren quartäre Ammonium- oder Phosphoniumsalze der allgemeinen Formel (I) eingesetzt werden,
(R¹R²R³R⁴A)⁺X⁻ (I)
worin
A für N oder P steht,
X⁻ für ein anorganisches oder organisches Anion steht,
R¹, R², R³ und R⁴ gleich oder verschieden sind und für Alkyl-, Aryl-, Aralkyl-, Cycloalkyl- oder Heteroaryl-Reste stehen, die durch einen oder mehrere, gleiche oder verschiedene Halogenreste substituiert sein können, oder aber jeweils zwei Reste unter Einbindung von A einen C₄-C₆-Cycloalkyl-Ring bilden können.

4. Verfahren nach Anspruch 3, **dadurch gekennzeichnet, dass** in der allgemeinen Formel (I) X für F⁻, Cl⁻, Br⁻, I⁻, OH⁻, NO₃⁻, HSO₄⁻, SO₄⁻, CH₃COO⁻, CF₃COO⁻, C₂H₅COO⁻, C₃H₇COO⁻, CF₃SO₃⁻ oder C₄F₉SO₃⁻ steht.

5. Verfahren nach einem oder mehreren der Ansprüche 2-4, **dadurch gekennzeichnet, dass** solche Phasentransferkatalysatoren der allgemeinen Formel (I) eingesetzt werden, bei denen R¹, R², R³ und R⁴ gleich oder verschieden sind und für C₁-C₁₈-Alkyl, C₆-C₁₈-Aryl, C₇-C₁₉-Aralkyl, C₅-C₇-Cycloalkyl oder C₃-C₁₈-Heteroaryl stehen.

6. Verfahren nach Anspruch 3, **dadurch gekennzeichnet, dass** als Phasentransferkatalysator ((C₄H₉)₄N)⁺Hal⁻, bevorzugt ((C₄H₉)₄N)⁺Br⁻, ((C₄H₉)₄P)⁺Hal⁻, bevorzugt ((C₄H₉)₄P)⁺Br⁻, ((C₄H₉)₄N)⁺HSO₄⁻, ((C₈H₁₇)₄N)⁺Br⁻, [(CH₃)(C₈H₁₇)₃N]⁺Cl⁻ oder [(CH₃)(C₄H₉)₃ N]⁺Cl⁻ eingesetzt werden.

7. Verfahren nach einem oder mehreren der Ansprüche 1-6, **dadurch gekennzeichnet, dass** der Phasentransferkatalysator in einer Menge im Bereich von 0.1-20 mol%, bevorzugt im Bereich von 0,5-15 mol%, besonders bevorzugt im Bereich von 0,5-11 mol%, jeweils bezogen auf das eingesetzte α,β-ungesättigte Enon oder α,β-ungesättigte Sulfon, eingesetzt wird.

8. Verfahren nach einem oder mehreren der Ansprüche 1-7, **dadurch gekennzeichnet, dass** als α,β-ungesättigte Enone oder α,β-ungesättigte Sulfone Verbindungen der allgemeinen Formel (II) 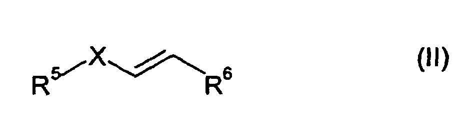 eingesetzt werden, worin
X für (C=O) oder (SO₂) steht und
R⁵ und R⁶ gleich oder verschieden sind und (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₇-C₁₉)-Aralkyl, (C₁-C₁₈)-Heteroaryl oder (C₂-C₁₉)-Heteroaralkyl bedeuten,
wobei die für R⁵ und R⁶ genannten Reste einfach oder mehrfach mit gleichen oder verschiedenen Resten Rest R⁷, Halogen, NO₂, NR⁷R⁸, PO₀₋₃R⁷R⁸, SO₀₋₃R⁷, OR⁷, ,CO₂R⁷, CONHR⁷ oder COR⁷ substituiert sein können und gegebenenfalls eine oder mehrere CH₂-Gruppen in den Resten R⁵ und R⁶ durch O, SO₀₋₂, NR⁷ oder PO₀₋₂R⁷ substituiert sein können,
wobei R⁷ und R⁸ gleich oder verschieden sind und H, (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₁-C₁₈)-Heteroaryl, (C₁-C₈)-Alkyl-(C₆-C₈)-Aryl, (C₁-C₈)-Alkyl-(C₁-C₁₈)-Heteroaryl, (C₁-C₈)-Alkyl-(C₃-C₈)-Cycloalkyl bedeuten und diese Reste R⁷ und R⁸ einfach oder mehrfach mit gleichen oder verschiedenen Halogenresten substituiert sein können.

9. Verfahren nach einem oder mehreren der Ansprüche 1-8, **dadurch gekennzeichnet, dass** als α,β-ungesättigte Enone oder α,β-ungesättigte Sulfone Verbindungen der allgemeinen Formel (II) eingesetzt werden, in denen R⁵ und R⁶ gleich oder verschieden sind und (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei die zuvor genannten Reste einfach oder mehrfach mit gleichen oder verschiedenen Resten R⁷, Halogen, NO₂, NR⁷R⁸, PO₀₋₃R⁷R⁸ oder OR⁷ substituiert sein können und R⁷ und R⁸ die für die allgemeine Formel (II) angegebenen Bedeutungen besitzen.

10. Verfahren nach einem oder mehreren der Ansprüche 1-9, **dadurch gekennzeichnet, dass** als α,β-ungesättigte Enone oder α,β-ungesättigte Sulfone Verbindungen der allgemeinen Formel (II) eingesetzt werden, in denen R⁵ und R⁶ gleich oder verschieden sind und (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei die zuvor genannten Reste einfach oder mehrfach mit gleichen oder verschiedenen Resten R⁷, Halogen, NO₂, NR⁷R⁸, PO₀₋₃R⁷R⁸ oder OR⁷ substituiert sein können und R⁷ und R⁸ die für die allgemeine Formel (II) zuvor angegebene Bedeutungen besitzen, mit der Maßgabe, dass mindestens einer der Reste R⁵ oder R⁶ einen (C₂-C₁₂)-Alkenyl-, (C₂-C₁₂)-Alkinyl-, (C₆-C₁₂)-Aryl- oder (C₁-C₁₂)-Heteroarylrest darstellt.

11. Verfahren nach einem oder mehreren der Ansprüche 1-10, **dadurch gekennzeichnet, dass** als α,β-ungesättigte Enone oder α,β-ungesättigte Sulfone Verbindungen der allgemeinen Formel (III) eingesetzt werden, 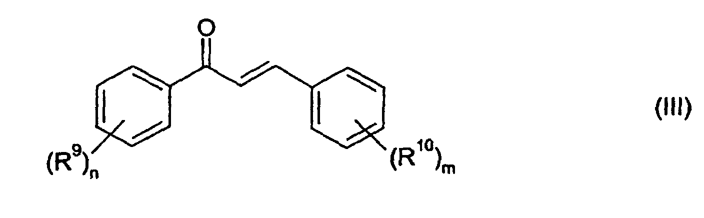 wobei
n und m gleich oder verschieden sind und für die Zahlen 0, 1, 2 oder 3 stehen und
R⁹ und R¹⁰ gleich oder verschieden sind und NR⁷R⁸, NO₂, OR⁷, (C₁-C₁₂)-Alkyl, (C₂-C₁₂)-Alkenyl, (C₂-C₁₂)-Alkinyl, (C₅-C₈)-Cycloalkyl, (C₆-C₁₂)-Aryl oder (C₁-C₁₂)-Heteroaryl bedeuten, wobei diese Reste R⁹ und R¹⁰ einfach oder mehrfach mit gleichen oder verschiedenen Halogenresten substituiert sein können und R ⁷ und R⁸ die für Formel (II) genannten Bedeutungen besitzen.

12. Verfahren nach einem oder mehreren der Ansprüche 1-11, **dadurch gekennzeichnet, dass** als diastereomeren- und enantiomerenangereicherten Homo-Polyaminosäuren solche aus der Gruppe Polyneopentylglycin, Polyleucin, Polyisoleucin, Polyvalin, Polyalanin und Polyphenylalanin verwendet werden.

13. Verfahren nach einem oder mehreren der Ansprüche 1-12, **dadurch gekennzeichnet, dass** die Kettenlänge der Polyaminosäuren im Bereich von 5 bis 100, vorzugsweise im Bereich von 7 bis 50 und insbesondere im Bereich von 10 bis 40 Aminosäure-Wiederholungseinheiten liegt.

14. Verfahren nach einem oder mehreren der Ansprüche 1-13, **dadurch gekennzeichnet, dass** die Homo-Polyaminosäure im Bereich von 0.0001-40 mol%, bevorzugt im Bereich von 0.001-20 mol%, besonders bevorzugt im Bereich von 0.01-15 mol%, und insbesondere im Bereich von 1 bis 15 mol-% jeweils bezogen auf das eingesetzte α,β-ungesättigte Enon oder α,β-ungesättigte Sulfon, eingesetzt wird.

15. Verfahren nach einem oder mehreren der Ansprüche 1-14, **dadurch gekennzeichnet, dass** man Homo-Polyaminosäuren einsetzt, die adsorptiv auf einem unlöslichen Trägermaterial, bevorzugt solchen auf Siliziumdioxid- oder Zeolith-Basis, auf Aktivkohle oder auf Zuckerderivate, bevorzugt Nitrocellulose oder Cellulose, aufgebracht sind.

16. Verfahren nach einem oder mehreren der Ansprüche 1-15, **dadurch gekennzeichnet, dass** als Oxidationsmittel Wasserstoffperoxid-Komplexe mit anorganischen Carbonaten, tertiären Aminen, Aminoxiden, Amiden, Phosphanen oder Phosphanoxiden eingesetzt werden, bevorzugt ein Harnstoff-Wasserstoffperoxid-Komplex.

17. Verfahren nach einem oder mehreren der Ansprüche 1-16, **dadurch gekennzeichnet, dass** das Oxidationsmittel in einer Menge von 1-10 Äquivalenten, bevorzugt im Bereich von 1-5 Äquivalenten, besonders bevorzugt von 1-3 Äquivalenten und insbesondere von 1-2 Äquivalenten eingesetzt wird.

18. Verfahren nach einem oder mehreren der Ansprüche 1-17, **dadurch gekennzeichnet, dass** als Basen organische oder anorganische Basen, bevorzugt organische, nicht-nucleophile Basen, insbesondere DBU (1,8-Diazabicyclo[5.4.0]undec-7-en), DBN (1,5-Diazabicyclo[4.3.0]non-5-en) oder DABCO (1,4-Diazabicyclo[2.2.2]octan) eingesetzt werden.

19. Verfahren nach einem oder mehreren der Ansprüche 1-18, **dadurch gekennzeichnet, dass** die Base in einer Menge von 0,1-10 Äquivalenten, bevorzugt von 0,5-5 Äquivalenten, besonders bevorzugt von 0,8-2 Äquivalenten eingesetzt wird.

20. Verfahren nach einem oder mehreren der Ansprüche 1-19, **dadurch gekennzeichnet, dass** als organisches Lösungsmittel Ether, bevorzugt THF, Diethylether oder *tert*.-Butylmethylether, Ester, bevorzugt Ethylacetat, Amide, bevorzugt Dimethylformamid, oder Sulfoxide, bevorzugt Dimethylsulfoxid eingesetzt werden.

21. Verfahren nach einem oder mehreren der Ansprüche 1-20, **dadurch gekennzeichnet, dass** die Temperatur, die bei der Epoxidierung verwendet wird, im Bereich von -10 bis +50 °C, bevorzugt im Bereich von 0 bis +40 °C und insbesondere im Bereich von +10 bis +30 °C liegt.

## Claims

1. Process for the epoxidation of α,β-unsaturated enones or α,β-unsaturated sulphones in the presence of
(1) an organic solvent
(2) a base,
(3) an oxidant,
(4) a diastereomer- and enantiomer-enriched homo-polyamino acid as catalyst, which has not been separately preactivated,
**characterized in that** the epoxidation is
(5) carried out in the presence of a phase-transfer catalyst and without addition of water.

2. Process according to Claim 1, **characterized in that** quaternary ammonium salts, quaternary phosphonium salts, onium compounds or pyridinium salts are employed as phase-transfer catalysts.

3. Process according to Claim 2, **characterized in that** the phase-transfer catalysts employed are quaternary ammonium or phosphonium salts of the general formula (I).
(R¹R²R³R⁴A)⁺X⁻ (I)
in which
A is N or P,
X⁻ is an inorganic or organic anion,
R¹, R², R³ and R⁴ are identical or different and are alkyl, aryl, aralkyl, cycloalkyl or heteroaryl radicals which may be substituted by one or more identical or different halogen radicals, or else two radicals in each case may form a C₄-C₆-cycloalkyl ring including A.

4. Process according to Claim 3, **characterized in that** X in the general formula (I) is F⁻, Cl⁻, Br⁻, I⁻, OH⁻, NO₃⁻, HSO₄⁻, SO₄⁻, CH₃COO⁻, CF₃COO⁻, C₂H₅COO⁻, C₃H₇COO⁻, CF₃SO₃⁻ or C₄F₉SO₃⁻.

5. Process according to one or more of Claims 2-4, **characterized in that** phase-transfer catalysts of the general formula (I) in which R¹, R², R³ and R⁴ are identical or different and are C₁-C₁₈-alkyl, C₆-C₁₈-aryl, C₇-C₁₉-aralkyl, C₅-C₇-cycloalkyl or C₃-C₁₈-heteroaryl are employed.

6. Process according to Claim 3, **characterized in that** ((C₄H₉)₄N)⁺Hal⁻, preferably ((C₄H₉)₄N)⁺Br⁻, ((C₄H₉)₄P)⁺Hal⁻, preferably ((C₄H₉)₄P)⁺Br⁻, ((C₄H₉)₄N)⁺HSO₄⁻, ((C₈H₁₇)₄N)⁺Br⁻, [(CH₃)(C₈H₁₇)₃N]⁺Cl⁻ or [(CH₃)(C₄H₉)₃N]⁺Cl⁻ are employed as phase-transfer catalyst.

7. Process according to one or more of Claims 1-6, **characterized in that** the phase-transfer catalyst is employed in an amount in the range 0.1-20 mol%, preferably in the range 0.5-15 mol%, particularly preferably in the range 0.5-11 mol%, in each case based on the α,β-unsaturated enone or α,β-unsaturated sulphone employed.

8. Process according to one or more of Claims 1-7, **characterized in that** the compounds employed as α,β-unsaturated enones or α,β-unsaturated sulphones have the general formula (II) 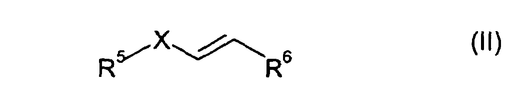 in which
X is (C=O) or (SO₂), and
R⁵ and R⁶ are identical or different and are (C₁-C₁₈)-alkyl, (C₂-C₁₈)-alkenyl, (C₂-C₁₈)-alkynyl, (C₃-C₈)-cycloalkyl, (C₆-C₁₈)-aryl, (C₇-C₁₉)-aralkyl, (C₁-C₁₈)-heteroaryl or (C₂-C₁₉)-heteroaralkyl,
where the radicals mentioned for R⁵ and R⁶ may be substituted once or more than once by identical or different radicals R⁷, halogen, NO₂, NR⁷R⁸, PO₀₋₃R⁷R⁸, SO₀₋₃R⁷, OR⁷, CO₂R⁷, CONHR⁷ or COR⁷, and optionally one or more CH₂ groups in the radicals R⁵ and R⁶ are replaced by O, SO₀₋₂, NR⁷ or PO₀₋₂R⁷,
where R⁷ and R⁸ are identical or different and are H, (C₁-C₁₈)-alkyl, (C₂-C₁₈)-alkenyl, (C₂-C₁₈)-alkynyl, (C₃-C₈)-cycloalkyl, (C₆-C₁₈)-aryl, (C₁-C₁₈)-heteroaryl, (C₁-C₈)-alkyl-(C₆-C₈)-aryl, (C₁-C₈)-alkyl-(C₁-C₁₈)-heteroaryl, (C₁-C₈)-alkyl-(C₃-C₈)-cycloalkyl, and these radicals R⁷ and R⁸ may be substituted once or more than once by identical or different halogen radicals.

9. Process according to one or more of Claims 1-8, **characterized in that** the compounds employed as α,β-unsaturated enones or α,β-unsaturated sulphones have the general formula (II) in which R⁵ and R⁶ are identical or different and are (C₁-C₁₂)-alkyl, (C₂-C₁₂)-alkenyl, (C₂-C₁₂)-alkynyl, (C₅-C₈)-cycloalkyl, (C₆-C₁₂)-aryl or (C₁-C₁₂)-heteroaryl, where the aforementioned radicals may be substituted once or more than once by identical or different radicals R⁷, halogen, NO₂, NR⁷R⁸, PO₀₋₃R⁷R⁸ or OR⁷, and R⁷ and R⁸ have the meanings indicated above for the general formula (II).

10. Process according to one or more of Claims 1-9, **characterized in that** the α,β-unsaturated enones or α,β-unsaturated sulphones employed are compounds of the general formula (II) in which R⁵ and R⁶ are identical or different and are (C₁-C₁₂)-alkyl, (C₂-C₁₂)-alkenyl, (C₂-C₁₂)-alkynyl, (C₅-C₈)-cycloalkyl, (C₆-C₁₂)-aryl or (C₁-C₁₂)-heteroaryl, where the aforementioned radicals may be substituted once or more than once by identical or different radicals R⁷, halogen, NO₂, NR⁷R⁸, PO₀₋₃R⁷R⁸ or OR⁷, and R⁷ and R⁸ have the meanings indicated above for the general formula (II), with the proviso that at least one of the radicals R⁵ or R⁶ is a (C₂-C₁₂)-alkenyl, (C₂-C₁₂)-alkynyl, (C₆-C₁₂)-aryl- or (C₁-C₁₂)-heteroaryl radical.

11. Process according to one or more of Claims 1-10, **characterized in that** the α,β-unsaturated enones or α,β-unsaturated sulphones employed are compounds of the general formula (III) 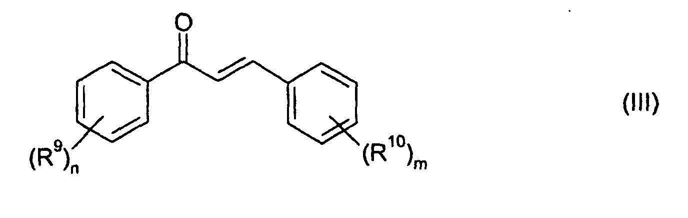 where
n and m are identical or different and are the numbers 0, 1, 2 or 3,
and
R⁹ and R¹⁰ are identical or different and are NR⁷R⁸, NO₂, OR⁷, (C₁-C₁₂)-alkyl, (C₂-C₁₂)-alkenyl, (C₂-C₁₂)-alkynyl, (C₅-C₈)-cycloalkyl, (C₆-C₁₂)-aryl or (C₁-C₁₂)-heteroaryl where these radicals R⁹ and R¹⁰ may be substituted once or more than once by identical or different halogen radicals, and R ⁷ and R⁸ have the meanings mentioned for formula (II).

12. Process according to one or more of Claims 1-11, **characterized in that** the diastereomer- and enantiomer-enriched homo-polyamino acids used are those from the group of polyneopentylglycine, polyleucine, polyisoleucine, polyvaline, polyalanine and polyphenylalanine.

13. Process according to one or more of Claims 1-12, **characterized in that** the chain length of the polyamino acids is in the range from 5 to 100, preferably in the range from 7 to 50, and in particular in the range from 10 to 40, amino acid repeating units.

14. Process according to one or more of Claims 1-13, **characterized in that** the homo-polyamino acids are employed in the range 0.0001-40 mol%, preferably in the range 0.001-20 mol%, particularly preferably in the range 0.01-15 mol% and in particular in the range 1 to 15 mol %, in each case based on the α,β-unsaturated enone or α,β-unsaturated sulphone employed.

15. Process according to one or more of Claims 1-14, **characterized in that** homo-polyamino acids which are applied by adsorption to an insoluble support material, preferably those based on silica or zeolite, or activated carbon or sugar derivatives, preferably nitrocellulose or cellulose are used.

16. Process according to one or more of Claims 1-15, **characterized in that** hydrogen peroxide complexes with inorganic carbonates, tertiary amines, amino oxides, amides, phosphanes or phosphane oxides are employed as oxidant, preferably a urea/hydrogen peroxide complex.

17. Process according to one or more of Claims 1-16, **characterized in that** the oxidant is employed in an amount of 1-10 equivalents, preferably in the range of 1-5 equivalents, particularly preferably of 1-3 equivalents and in particular 1-2 equivalents.

18. Process according to one or more of Claims 1-17, **characterized in that** the bases employed are oganic or inorganic bases, preferably organic, non-nucleophilic bases, in particular DBU (1,8-diazabicyclo[5.4.0]undec-7-ene), DBN (1,5-diazabicyclo[4.3.0]non-5-ene) or DABCO (1,4-Diazabicyclo[2.2.2]octane).

19. Process according to one or more of Claims 1-18, **characterized in that** the base is employed in an amount of 0.1-10 equivalents, preferably of 0.5-5 equivalents, particularly preferably of 0.8-2 equivalents.

20. Process according to one or more of Claims 1-19, **characterized in that** ethers, preferably THF, diethyl ether or *tert*-butyl methyl ether, esters, preferably ethyl acetate, amides, preferably dimethylformamide, or sulphoxides, preferably dimethyl sulphoxide, are employed as organic solvent.

21. Process according to one or more of Claims 1-20, **characterized in that** the temperature which is used in the epoxidation is in the range from -10 to +50°C, preferably in the range from 0 to +40°C and in particular in the range from +10 to +30°C.

## Revendications

1. Procédé pour l'époxydation d'énones α,β-insaturées ou de sulfones α,β-insaturées en présence de
(1) un solvant organique,
(2) une base,
(3) un agent oxydant,
(4) un homo-polyaminoacide enrichi en diastéréo-isomères et en énantiomères, qui n'a pas subi d'activation préalable séparée, et qui sert de catalyseur,
**caractérisé en ce que** l'époxydation est réalisée
(5) en présence d'un catalyseur à transfert de phase et sans adjonction d'eau.

2. Procédé selon la revendication 1, **caractérisé en ce que** l'on utilise en tant que catalyseurs à transfert de phase des sels d'ammonium quaternaires, des sels de phosphonium quaternaires, des dérivés en onium ou des sels de pyridinium.

3. Procédé selon la revendication 2, **caractérisé en ce que** l'on utilise en tant que catalyseurs à transfert de phase des sels d'ammonium ou de phosphonium quaternaires de formule générale (I)
(R¹R²R³R⁴A)⁺X⁻ (I)
dans laquelle
A représente N ou P,
X⁻ représente un anion minéral ou organique,
R¹, R², R³ et R⁴, ayant des significations identiques ou différentes, représentent chacun un groupe alkyle, aryle, aralkyle, cycloalkyle ou hétéroaryle, chacun d'eux pouvant porter un ou plusieurs substituants halogéno identiques ou différents, ou bien deux de ces groupes peuvent former, avec A, un noyau cycloalkyle en C₄-C₆.

4. Procédé selon la revendication 3, **caractérisé en ce que**, dans la formule (I), X représente F⁻, Cl⁻, Br⁻, I⁻, OH⁻, NO₃⁻, HSO₄⁻, SO₄⁻, CH₃COO⁻, CF₃COO⁻, C₂H₅COO⁻, C₃H₇COO⁻, CF₃SO₃⁻ ou C₄F₉SO₃⁻.

5. Procédé selon une ou plusieurs des revendications 2 à 4, **caractérisé en ce que** l'on utilise des catalyseurs à transfert de phase de formule générale (I) dans laquelle R¹, R², R³ et R⁴, ayant des significations identiques ou différentes, représentent chacun un groupe alkyle en C₁-C₁₈, aryle en C₆-C₁₈, aralkyle en C₇-C₁₉, cycloalkyle en C₅-C₇ ou hétéroaryle en C₃-C₁₈.

6. Procédé selon la revendication 3, **caractérisé en ce que** l'on utilise en tant que catalyseurs à transfert de phase ((C₄H₉)₄N)⁺Hal⁻, de préférence ((C₄H₉)₄N)⁺Br⁻, ((C₄H₉)₄P)⁺Hal⁻, de préférence ((C₄H₉)₄P)⁺Br⁻, ((C₄H₉)₄N)⁺HSO₄⁻, ((C₈H₁₇)₄N)⁺Br⁻, [(CH₃)(C₈H₁₇)₃N] ⁺Cl⁻ ou [(C₃)(C₄H₉)₃N]⁺Cl⁻.

7. Procédé selon une ou plusieurs des revendications 1 à 6, **caractérisé en ce que** le catalyseur à transfert de phase est mis en oeuvre en quantité de 0,1 à 20 mol %, de préférence de 0,5 à 15 mol % et plus spécialement de 0,5 à 11 mol %, dans tous les cas par rapport à l'énone α,β-insaturée ou à la sulfone α,β-insaturée mise en oeuvre.

8. Procédé selon une ou plusieurs des revendications 1 à 7, **caractérisé en ce que** l'on met en oeuvre en tant qu'énones α,β-insaturées ou sulfones α,β-insaturées des composés de formule générale (II) 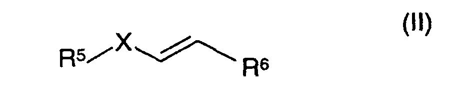 dans laquelle
X représente (C=O) ou (SO₂) et
R⁵ et R⁶, ayant des significations identiques ou différentes, représentent chacun un groupe alkyle en C₁-C₁₈, alcényle en C₂-C₁₈, alcynyle en C₂-C₁₈, cycloalkyle en C₃-C₈, aryle en C₆-C₁₈, aralkyle en C₇-C₁₉, hétéroaryle en C₁-C₁₈ ou hétéroaralkyle en C₂-C₁₉,
chacun de ces groupes pouvant porter un ou plusieurs substituants identiques ou différents choisis parmi R⁷, les halogènes, NO₂, NR⁷R⁸, PO₀₋₃R⁷R⁸, SO₀₋₃R⁷, OR⁷, CO₂R⁷, CONHR⁷ ou COR⁷ et le cas échéant un ou plusieurs groupes CH₂ des groupes R⁵ et R⁶ peuvent être remplacés par O, SO₀₋₂, NR⁷ ou PO₀₋₂R⁷,
R⁷ et R⁸, ayant des significations identiques ou différentes, représentent chacun H, un groupe alkyle en C₁-C₁₈, alcényle en C₂-C₁₈, alcynyle en C₂-C₁₈, cycloalkyle en C₃-C₈, aryle en C₆-C₁₈, hétéroaryle en C₁-C₁₈, (alkyle en C₁-C₈)-aryle en C₆-C₈, (alkyle en C₁-C₈)-hétéroaryle en C₁-C₁₈, (alkyle en C₁-C₈)-cycloalkyle en C₃-C₈), chacun de ces groupes R⁷ et R⁸ pouvant encore porter un ou plusieurs substituants halogéno identiques ou différents.

9. Procédé selon une ou plusieurs des revendications 1 à 8, **caractérisé en ce que** l'on met en oeuvre des énones α,β-insaturées ou sulfones α,β-insaturées de formule générale (II) dans laquelle R⁵ et R⁶, ayant des significations identiques ou différentes, représentent chacun un groupe alkyle en C₁-C₁₂, alcényle en C₂-C₁₂, alcynyle en C₂-C₁₂, cycloalkyle en C₅-C₈, aryle en C₆-C₁₂ ou hétéroaryle en C₁-C₁₂, chacun de ces groupes pouvant porter un ou plusieurs substituants identiques ou différents choisis parmi R⁷, les halogènes, NO₂, NR⁷R⁸, PO₀₋₃R⁷R⁸ ou OR⁷, R⁷ et R⁸ ayant les significations indiquées en référence à la formule générale (II).

10. Procédé selon une ou plusieurs des revendications 1 à 9, **caractérisé en ce que** l'on met en oeuvre des énones α,β-insaturées ou des sulfones α,β-insaturées de formule générale (II) dans laquelle R⁵ et R⁶, ayant des significations identiques ou différentes, représentent chacun un groupe alkyle en C₁-C₁₂, alcényle en C₂-C₁₂, alcynyle en C₂-C₁₂, cycloalkyle en C₅-C₈, aryle en C₆-C₁₂ ou hétéroaryle en C₁-C₁₂, chacun d'eux pouvant porter un ou plusieurs substituants identiques ou différents choisis parmi R⁷, les halogènes, NO₂, NR⁷R⁸, PO₀₋₃R⁷R⁸ ou OR⁷, R⁷ et R⁸ ayant les significations indiquées ci-dessus en référence à la formule générale (II), sous réserve que l'un au moins des symboles R⁵ ou R⁶ représente un groupe alcényle en C₂-C₁₂, alcynyle en C₂-C₁₂, aryle en C₆-C₁₂ ou hétéroaryle en C₁-C₁₂.

11. Procédé selon une ou plusieurs des revendications 1 à 10, **caractérisé en ce que** l'on met en oeuvre des énones α,β-insaturées ou des sulfones α,β-insaturées de formule générale (III) 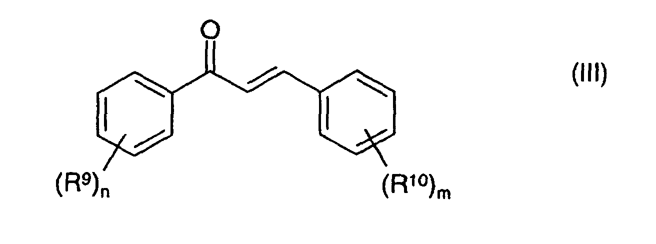 dans laquelle
n et m, identiques ou différents, sont égaux chacun à 0, 1, 2 ou 3,
R⁹ et R¹⁰, ayant des significations identiques ou différentes, représentent chacun NR⁷R⁸, NO₂, OR⁷, un groupe alkyle en C₁-C₁₂, alcényle en C₂-C₁₂, alcynyle en C₂-C₁₂, cycloalkyle en C₅-C₈, aryle en C₆-C₁₂ ou hétéroaryle en C₁-C₁₂, chacun de ces groupes pouvant porter un ou plusieurs substituants halogéno identiques ou différents, et R⁷ et R⁸ ayant les significations indiquées en référence à la formule générale (II).

12. Procédé selon une ou plusieurs des revendications 1 à 11, **caractérisé en ce que** l'on utilise en tant qu'homo-polyaminoacides enrichis en diastéréo-isomères et en énantiomères des composés du groupe de la polynéopentylglycine, de la polyleucine, de la polyisoleucine, de la polyvaline, de la polyalanine et de la polyphénylalanine.

13. Procédé selon une ou plusieurs des revendications 1 à 12, **caractérisé en ce que** la longueur de chaîne des polyaminoacides se situe dans l'intervalle de 5 à 100, de préférence de 7 à 50 et plus spécialement de 10 à 40 motifs répétés d'aminoacides.

14. Procédé selon une ou plusieurs des revendications 1 à 13, **caractérisé en ce que** l'homo-polyaminoacide est mis en oeuvre en quantité de 0,0001 à 40 mol %, de préférence de 0,001 à 20 mol %, plus spécialement de 0,01 à 15 mol % et dans les meilleures conditions de 1 à 15 mol % dans tous les cas par rapport à l'énone α,β-insaturée ou la sulfone α,β-insaturée mise en oeuvre.

15. Procédé selon une ou plusieurs des revendications 1 à 14, **caractérisé en ce que** l'on utilise des homo-polyaminoacides appliqués par adsorption sur une matière de support insoluble, de préférence à base de silice ou de zéolite, sur charbon actif ou sur des dérivés de sucres, de préférence la nitrocellulose ou la cellulose.

16. Procédé selon une ou plusieurs des revendications 1 à 15, **caractérisé en ce que** l'on utilise en tant qu'agents oxydants des complexes du peroxyde d'hydrogène et de carbonates minéraux, d'amines tertiaires, d'oxydes d'amines, d'amides, de phosphanes ou d'oxydes de phosphane, de préférence un complexe d'urée-peroxyde d'hydrogène.

17. Procédé selon une ou plusieurs des revendications 1 à 16, **caractérisé en ce que** l'agent oxydant est mis en oeuvre en quantité de 1 à 10 équivalents, de préférence de 1 à 5 équivalents, plus spécialement de 1 à 3 équivalents et dans les meilleures conditions de 1 à 2 équivalents.

18. Procédé selon une ou plusieurs des revendications 1 à 17, **caractérisé en ce que** l'on utilise en tant que bases des bases minérales ou organiques, de préférence des bases organiques non nucléophiles, en particulier le DBU (1,8-diazabicyclo[5.4.0]undéc-7-ène), le DBN (1,5-diazabicyclo[4.3.0]non-5-ène) ou le DABCO (1,4-diazabicyclo[2.2.2]-octane).

19. Procédé selon une ou plusieurs des revendications 1 à 18, **caractérisé en ce que** la base est mise en oeuvre en quantité de 0,1 à 10 équivalents, de préférence de 0,5 à 5 équivalents et plus spécialement de 0,8 à 2 équivalents

20. Procédé selon une ou plusieurs des revendications 1 à 19, **caractérisé en ce que** l'on utilise en tant que solvants organiques des éthers, de préférence le tétrahydrofuranne, l'éther diéthylique ou l'oxyde de méthyle ou de tert-butyle, des esters, de préférence l'acétate d'éthyle, des amides, de préférence le diméthylformamide ou des sulfoxydes, de préférence le diméthylsulfoxyde.

21. Procédé selon une ou plusieurs des revendications 1 à 20, **caractérisé en ce que** l'on réalise l'époxydation à une température située dans l'intervalle de -10 à +50°C, de préférence de 0 à +40°C et plus spécialement de +10 à +30°C.
